# EUROPEAN PATENT SPECIFICATION

(11) **EP 0 810 847 B1**
(45) Date of publication and mention of the grant of the patent: **16.08.2000**
(21) Application number: 96904363.7
(22) Date of filing: 21.02.1996
(51) Int. Cl.: A61F 13/42, A61F 5/48

(54) **ASSEMBLY FOR DETECTING AND SIGNALIZING WETNESS**
SYSTEM ZUR FESTSTELLUNG UND ZUM ANZEIGEN VON FEUCHTIGKEIT
ENSEMBLE DETECTEUR-INDICATEUR D'HUMIDITE

(30) Priority: 22.02.1995 NL 9500338
(43) Date of publication of application: 10.12.1997
(73) Proprietor: Van Twist, Wilhelmus Theodorus Franciscus, 4741 TJ Hoeven (NL)
(72) Inventor: Van Twist, Wilhelmus Theodorus Franciscus, 4741 TJ Hoeven (NL)
(74) Representative: Lips, Hendrik Jan George, Ir.
(86) International application number: NL9600083
(87) International publication number: WO9625904

(56) References cited:
- WO-A-94/07224
- WO-A-95/15739
- DE-A- 3 440 584
- GB-A- 2 250 121
- US-A- 5 036 859
- US-A- 5 266 928

## Description

The invention relates to an assembly for detecting and signalizing wetness, said assembly comprising an electronic device being mounted in a housing and is provided with contacts for establishing the connection between said device and two elongated conductors being part of a sensor strip and being fixedly mounted at mutual distance on a conductor bearing strip from a material being non-conductive in a dry state, said sensor strip being provided with a layer of adhesive for adhering it to a diaper or similar object, in such a way that on the presence of wetness near the conductors, the electronic device will produce a signal, and the sensor strip can be disposed after use.

Such an assembly is known from US-A-5.226.928. Here, the adhesive is applied to the other side of the conductor bearing strip than the one on which the conductors are situated. When the sensor strip is adhered in a diaper, the conductors will thus lie against the skin of the diaper user.

Further, in this known assembly, the electronic device is placed between two strips from a flexible material being connected to each other for forming a flexible band. One strip is provided with pins projecting through the strip. At one side, said pins engage contacts of the electronic device and at the other side they engage the conductors of the sensor strip being pressed against the outside of the strip concerned, by means of an adhesive patch. The possibility of mounting the electronic device in a housing from plastic was indicated too. Then, the conductor bearing strip is provided with a leg being square to it, with a layer of adhesive on it. The housing of the electronic device is adhered to said leg in such a way that it will come to lie between said leg and the outside of the diaper. Then, it will be secured with the help of the leg which is adhered on the diaper at both sides of the housing. In the first embodiment, a less stable first connection between the conductors and the electronic device will be obtained, while in the second embodiment, the sensor strip must also be provided with an additional leg, apart from the conductor bearing strip, as a result of which the use of the assembly will become more complicated and the disposable sensor strip will become more expensive.

The object of the invention is to remove these difficulties and to that end provides for, that the conductor bearing strip is provided with a layer of adhesive on the same side as where the conductors are situated.

When during use of the assembly, the sensor strip is adhered to the inside of a diaper, the conductor bearing strip will contact the skin of the user and the conductors will not contact it. Practice has shown, that, with a correct choice of the material for the conductor bearing strip, the response time of the device will not be adversely affected.

DE-A-3 440 584 describes a bandage through the material of which two elongated conductors pass. So the conductors are positioned in the material during the manufacturing of said bandage and are forming part of it. So after use of said bandage the conductors, mainly consisting of a metal, together with the bandage will be thrown away. So this citation does not concern a strip provided with conductors which strip can be applied on a bandage by the user of it and might be removed from said bandage after use.

Like with the present invention, EP-A-0 270 048 also uses a sensor strip constituted by an elongated conductor bearing strip having two conductors on it. Here, the sensor strip is connected to an electronic device being situated in a belt by means of lines extending through a belt. When the sensor strip is to be used, it is slid into a sleeve which will be disposed of after use. One face of the sleeve is provided with a layer of adhesive material. Application of the sensor strip in the sleeve can only take place easily if the sensor has a certain rigidity. The sleeve will have to be in close contact with the sensor strip in order to be able to guarantee its proper operation. After sliding in the sensor strip, the sleeve will also have to be properly flat for enabling its adhesion. After use, the sensor strip might be wet and will have to be cleaned before it is slid into a new sleeve. Further, one must check whether the proper side of the sensor strip is directed towards the user.

Finally, GB-A-2 250 121 describes a diaper provided with two cavities, with a sensor part that can be slid into one of said cavities, whereas in the inner layer of the diaper, both at its front side and at its back side, a further sensor part is securely mounted. This diaper must be produced in a very special way, since two sensor parts have to be securely received in the diaper.

According to a further development of the invention, the sensor strip can be adhered onto the outside of a diaper and the diaper can be provided with at least one opening which will be situated near the sensor strip, in which the housing of the electronic device will simultaneously be secured on the diaper by means of the sensor strip. The sensor strip will then be applied across one face of the housing and be adhered on it, whereupon the other face of the housing will be positioned against the outside of the diaper and the further part of the sensor strip will be adhered to said outside at both sides of the housing.

In order to ensure that on connecting the sensor strip to the electronic device, a good contact will be established between said device and the conductors of the sensor strip, the housing of the electronic device, or a part connected to it, can be provided with a recession having a width being adapted to the sensor strip width.

This will determine the position of the sensor strip in relation to the electronic device.

The diaper can be provided with the opening directly before use, but said opening could also be applied earlier, when producing the diaper. Possibly, the opening can be sealed off by means of an adhesive strip, so that the diaper can also be used for normal purposes without the risk of leakage.

It is also possible to make some openings or assemblies of openings in the diaper so that on use, one can choose the opening or the assembly being in the most advantageous position in the case concerned.

Although a diaper in particular has been discussed in the above, it will be obvious that the assembly described can also be used for many other purposes, than for use with a diaper only. It would additionally be possible, not to use the sensor for signalizing wetness, but for measuring the temperature on a certain location.

To enhance the convenience in wearing, the conductors of the conductor bearing strip can be provided with a permeable layer from a fabric or a like material.

This material must be easily permeable and/or removable when the connection between the sensor strip and the contacts of the electronic device is to be established.

There is the possibility of producing the sensor strip, consisting of the conductor bearing strip and the conductors, according to a continuous process and winding it onto a spool. One can then cut off the desired length of the sensor strip while taking into account the application thereof. For a baby, one can use a smaller length than for a bigger child. Naturally, the sensor strip can also be supplied in measured lengths.

Like with the known assembly, the electronic device will produce a signal when the sensor strip detects a certain degree of humidity.

In order to be able to apply the assembly of the sensor strip according to the present invention and the accompanying electronic device for various purposes, the electronic device can be provided with means for adjusting its sensitivity.

For example, in one case it will be important that a signal will already be produced on detection of a small quantity of wetness, so with a very small moistening of the flexible material of the sensor strip, such as with bed-wetting. In another case, a signal will have to be produced only after a larger amount of wetness has been released. Thus, the device can also be used for signalizing the release of wound fluid. With immobile patients, after signalizing a certain degree of moisture, measures can be taken immediately, as a result of which the risk of decubitus can be reduced considerably. It has appeared that due to this, the number of lay days in hospitals, nursing homes and the like, can be reduced considerably, as well as the nursings costs. For example, it is also possible that incontinent persons or their attendants can be warned when certain reception elements should be changed.

In many cases, the electronic device will be designed such, that it produces an audio signal. The loudness of this signal can be controlled by means mounted on the device. Further, it is possible to provide the device with a transmitter having a wireless connection to a receiver system. Owing to this, in hospitals, nursing homes and the like, one can employ a central signalizing point.

It is also possible to have the electronic device generate a vibration serving as a warning signal for the user. Such a device can e.g. be used by incontinent persons or persons having a spinal cord lesion being capable of replacing receiving elements themselves. Then, only that person will detect the signal.

Further, the electronic device can be provided with a control knob which can be used for checking whether both conductors of the sensor strip, after having connected it to the device, are in contact with the electronic parts of the device.

The invention is further explained by way of embodiments, illustrated in the drawing, in which:
Fig. 1 shows schematically a perspective view of an electronic device provided with a clamping device having a sensor strip mounted therein;
Fig. 2 shows schematically a perspective view of a diaper or plastic pants with the electronic device mounted thereon and a sensor strip mounted therein;
Fig. 3 shows schematically a side view of the device of Fig. 1 with sensor strip;
Fig. 4 shows the view on a sensor strip;
Fig. 5 shows schematically a perspective view of a diaper or plastic pants provided with an opening and having an assembly according to the invention mounted on the outside of the diaper near said opening;
Fig. 6 shows a plan view of a part of an assembly as shown in Fig. 5 mounted on the outside of a diaper or a like object; and
Fig. 7 shows a side view of Fig. 6.

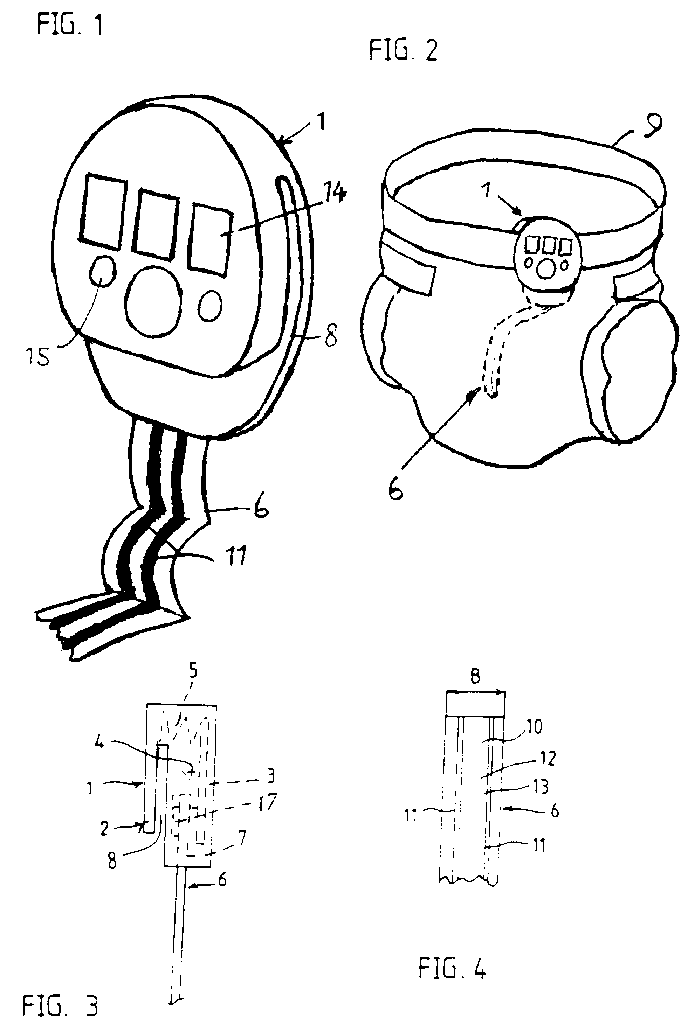

The electronic parts of the device 1 have not been indicated in the drawing, since they can be of very different designs. As illustrated in Fig. 3, the device 1 can comprise a fixed clamping part 2, in which the electric contacts 17 can project, and a clamping jaw 3 being pivotably around the pivot 4. By means of the spring 5, the clamping jaw 3 is brought to a position in which it can press a sensor strip 6 against the clamping portion 2. For a proper positioning of the sensor strip 6, the device 1 is provided with a recession 7 having a width being adapted to the width B of the sensor strip indicated in Fig. 4. Further, the device comprises the slot 8 so that it can be mounted on the edge of a diaper 9, see Fig. 2, or the like.

Mounting a device 1 having an sensor strip 6 on a diaper or plastic pants 9 is indicated in Fig. 2 in particular. It will be obvious that the length of the sensor strip 6 can be varied and that the sensor strip can be mounted at any location in the diaper, in such a way that it will produce the desired effect.

As appears from Fig. 4 in particular, the **sensor** strip 6 consists of a flexible conductor bearing strip 10 having one surface provided with two spaced apart conductors 11. The conductors 11 can be mounted by means of printing, for example. The conductors 11 can consist of various materials, such as from silver and copper, for example. Owing to this, a Volta effect can be generated. The conductors 11 can further be covered by a permeable layer 12 of fabric or like material. Additionally, an adhesive layer 13 can be applied on the layer 12 so that the sensor strip 6 can not move in relation to the device 1 and in relation to the diaper 9.

As appears from the Figs. 1 and 2, the device 1 can be provided with a number of knobs 14 and lights 15. The knobs can serve for adjusting the sensitivity of the device 1, for checking whether the conductors of a sensor strip contact the contacts of the device 1 and the like, for example.

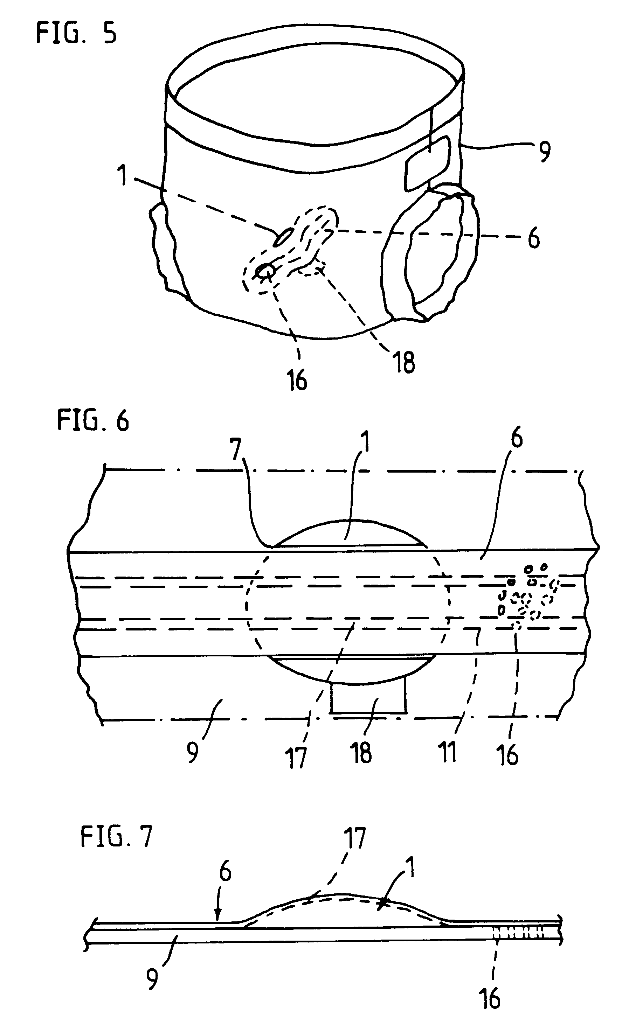

Fig. 5 shows in perspective view a diaper 9 in the form it will have in use. An assembly according to the present invention has been mounted on the outside of the diaper. Figs. 6 and 7 show in view and in cross-section, respectively, a part of the diaper 9 with the electronic device 1 of the assembly situated on its outside. This device 1 is provided with a recession 7 in which the sensor strip 6 is received. The recess accomodates the electric contacts that have to be in contact with the conductors 11 of the sensor strip 6 on using the assembly.

The sensor strip 6 is adhered to the outside of the diaper 9 as a result of which the electronic device 1 is secured simultaneously.

The diaper is provided with a number of openings 16. These openings 16 can have been made during production of the diaper, or immediately before the diaper will be used. Instead of a number of small openings, one could also make a single, larger opening, or some larger openings at some mutual distance. This will then take place during production of the diaper. The openings can then be taped up so that the diaper can also be used when the openings are not required. When the assembly according to the present invention has to be used, one or more openings can be cleared on the location which is the most suitable for that specific purpose.

As illustrated in Fig. 6, the electronic device 1 can be provided with a flexible tape 18, so that the device 1 can easily be removed from the sensor strip 6 after using it.

Although a diaper was discussed in the above, it was already stated that the invention can also be applied in use of a bandage or like objects.

It will be obvious, that only some possible embodiments of an assembly according to the invention have been illustrated in the drawing and described above, and that many changes can be made without leaving the inventive idea as it is indicated in the claims.

## Claims

1. Assembly for detecting and signalizing wetness, said assembly comprising an electronic device (1) being mounted in a housing and is provided with contacts (17) for establishing the connection between said electronic device (1) and two elongated conductors (11) being part of a sensor strip (6) and being fixedly mounted at mutual distance on a conductor bearing strip (10) from a material being non-conductive when in a dry state, said sensor strip (6) being provided with a layer of adhesive (13) for adhering it to a diaper (9) or similar object, in such a way that, on the presence of wetness near the conductors (11), the electronic device (1) will produce a signal and the sensor strip (6) can be disposed after use, characterized in that the conductor bearing strip (10) is provided with a layer of adhesive (13) on the same side as where the conductors (11) are situated.

2. Assembly according to claim 1, characterized in that the housing (2) of the electronic device (1), or a part (2, 3) connected to it, is provided with a recession (7) having its width adapted to the width (B) of the sensor strip (6), in which recession said sensor strip (6) can be positioned when using it such that the strip extends from both sides of the recession and the housing (2) can be secured on a diaper by means of said strip.

3. Assembly according to claims 1 or 2, characterized in that the conductors (11) of the conductor bearing strip (10) are provided with a permeable layer (12) from a fabric or a like material.

4. Assembly according to claim 3, characterized in that the sensor strip is produced in a continuous process together with the conductors (11) and is taken from a spool.

5. Use of the assembly according to claim 1 or 2, in a diaper or similar object characterized in that on its production, the diaper (7) is provided with at least one opening (16) or a number of openings.

6. Use according to claim 5, characterized in that the opening (16) or openings made in the diaper (9) is or are covered by an adhesive tape.

## Patentansprüche

1. System zur Feststellung und zum Anzeigen von Feuchtigkeit, wobei das System eine elektronische Einrichtung (1) umfaßt, die in einem Gehäuse angeordnet und mit Kontakten (17) versehen ist, um die Verbindung zwischen der elektronischen Einrichtung (1) und zwei langgestreckten Leitern (11) herzustellen, die Teil eines Sensorstreifens (6) sind und beabstandet auf einem Leitertragestreifen (10) fest angeordnet sind, dessen Werkstoff im trockenen Zustand nicht-leitend ist, wobei der Sensorstreifen (6) mit einer Klebstoffschicht (13) zum Ankleben an eine Windel oder ähnliches derart versehen ist, daß in Gegenwart von Feuchtigkeit nahe der Leiter (11) die elektronische Einrichtung (1) ein Signal abgibt und der Sensorstreifen (6) nach Gebrauch entsorgt werden kann,
**dadurch gekennzeichnet,**
daß der Leitertragestreifen (10) mit einer Klebstoffschicht (13) auf derselben Seite versehen ist, wo die Leiter (11) angeordnet sind.

2. System nach Anspruch 1, dadurch gekennzeichnet, daß das Gehäuse (2) der elektronischen Einrichtung (1), oder ein damit verbundener Teil (2, 3) mit einem Rezess (7) versehen ist, dessen Weite der Breite (B) des Sensorstreifens (6) angepaßt ist, in welchem Rezess der Sensorstreifen (6) angeordnet werden kann, wenn er benutzt wird, so daß der Streifen sich von beiden Seiten des Rezesses erstreckt und das Gehäuse (2) auf der Windel mit dem Streifen befestigt werden kann.

3. System nach Anspruch 1 oder 2, dadurch gekennzeichnet, daß die Leiter (11) des Leitertragestreifens (10) mit einer durchlässigen Schicht aus Gewebe oder dergleichen versehen sind.

4. System nach Anspruch 3, dadurch gekennzeichnet, daß der Sensorstreifen in einem kontinuierlichen Verfahren zusammen mit den Leitern (11) hergestellt und von einer Spule entnommen wird.

5. Einsatz des Systems nach Anspruch 1 oder 2, in einer Windel oder ähnlichem Gegenstand,
**dadurch gekennzeichnet,**
daß die Windel (7) bei ihrer Herstellung mit mindestens einer Öffnung (16) oder einer Anzahl von Öffnungen versehen wird.

6. Einsatz nach Anspruch 5, dadurch gekennzeichnet, daß die Öffnung (16) oder die Öffnungen in der Windel (9) mit einem Klebeband bedeckt ist/sind.

## Revendications

1. Ensemble pour détecter et signaler une humidité, ledit ensemble comprenant un dispositif électronique (1) monté dans un boîtier et pourvu de contacts (17) pour raccorder ledit dispositif électronique (1) et deux conducteurs allongés (11) faisant partie d'une bande détectrice (6) et montés, fixes, à distance l'un de l'autre sur une bande support de conducteurs (10) en un matériau non conducteur à l'état sec, ladite bande détectrice (6) étant pourvue d'une couche d'adhésif (13) pour adhérer sur une couche pour bébé (9) ou un objet similaire de telle manière que, en la présence d'humidité au voisinage des conducteurs (11), le dispositif électronique (1) émet un signal et on peut jeter la bande détectrice (6) après utilisation, caractérisé en ce que la bande support de conducteurs (10) est pourvue d'une couche d'adhésif (13) sur le même côté que celui sur lequel sont situés les conducteurs (11).

2. Ensemble selon la revendication 1, caractérisé en ce que le boîtier (2) du dispositif électronique (1), ou une partie (2, 3) qui lui est raccordée, comporte un évidement (7) dont la largeur est adaptée à la largeur (B) de la bande détectrice (6), ladite bande détectrice (6) pouvant être positionnée dans cet évidement lors de son utilisation de façon à s'étendre des deux côtés de l'évidement et le boîtier (2) pouvant être ainsi fixé sur une couche au moyen de ladite bande.

3. Ensemble selon la revendication 1 ou la revendication 2, caractérisé en ce que les conducteurs (11) de la bande support de conducteurs (10) sont pourvus d'une couche perméable (12) en tissu ou en matériau analogue.

4. Ensemble selon la revendication 3, caractérisé en ce que la bande détectrice est produite en un processus continu ensemble avec les conducteurs (11) et est prélevée d'une bobine.

5. Utilisation d'un ensemble selon la revendication 1 ou la revendication 2 dans une couche pour bébé ou un objet similaire, caractérisée en ce que, lors de sa production, la couche pour bébé (7) est pourvue d'au moins une ouverture (16) ou d'un certain nombre d'ouvertures.

6. Utilisation selon la revendication 5, caractérisée en ce que l'ouverture ou les ouvertures (16) faite(s) dans la couche pour bébé (9) est (sont) recouverte(s) par un ruban adhésif.
